(19) Europäisches Patentamt / European Patent Office / Office européen des brevets

(11) **EP 3 091 953 B1**

(12) **EUROPEAN PATENT SPECIFICATION**

(45) Date of publication and mention
of the grant of the patent:
**21.05.2025 Bulletin 2025/21**

(21) Application number: **15700824.4**

(22) Date of filing: **06.01.2015**

(51) International Patent Classification (IPC):
***A61F 11/08*** *(2006.01)*

(52) Cooperative Patent Classification (CPC):
**A61F 11/08**

(86) International application number:
**PCT/US2015/010265**

(87) International publication number:
**WO 2015/105774 (16.07.2015 Gazette 2015/28)**

(54) **MOLDED FOAM PUSH-TO-FIT EARPLUG AND METHOD**

GEFORMTER PUSH-TO-FIT OHRSTÖPSEL AUS SCHAUMSTOFF UND VERFAHREN

BOUCHON D'OREILLE A AJUSTEMENT INSTANTANE ET PROCEDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.01.2014 US 201461925770 P**

(43) Date of publication of application:
**16.11.2016 Bulletin 2016/46**

(73) Proprietor: **3M Innovative Properties Company**
**St. Paul, MN 55133-3427 (US)**

(72) Inventors:
• **TEETERS, Kenneth F.**
**Saint Paul, Minnesota 55133-3427 (US)**
• **HAMER, Jeffrey L.**
**Saint Paul, Minnesota 55133-3427 (US)**

(74) Representative: **Mathys & Squire**
**Theatinerstraße 7**
**80333 München (DE)**

(56) References cited:
**EP-A2- 0 108 728      WO-A1-2007/044766**
**WO-A1-98/06362      US-A- 5 573 015**

**Description**

**Technical Field**

This description relates to a hearing protection device, in particular a push-to-fit type earplug made entirely of a closed-cell, slow-recovery foam as well as methods of making such an earplug.

**Background**

The use of hearing protective and noise attenuating devices are well known, and various types of devices have been considered. Such devices include earplugs and semi-aural devices including foam or rubber materials that are inserted into, or placed over, the ear canal of a user to physically obstruct the passage of sound waves into the inner ear.
Compressible or "roll-down" type earplugs generally comprise a compressible, resilient body portion and may be made of suitable slow recovery foam materials. The earplug may be inserted into the ear canal of a user by first rolling it between fingers to compress the body portion, then pushing the body portion into the ear canal, and subsequently allowing the body portion to expand to fill the ear canal.
Push-to-fit type earplugs have also been considered, and may include a compressible attenuating portion and a stiff portion that extends from the attenuating portion. To insert a push-to-fit type earplug, the user grasps the stiff portion and pushes the attenuating portion into the ear canal with an appropriate level of force. The attenuating portion compresses as it is accommodated in the ear canal. Push-to-fit type earplugs may allow the earplug to be quickly and easily inserted in an ear canal, and may promote hygiene by minimizing contact with the attenuating portion of the earplug prior to insertion.
WO 2007/0044766 describes an earplug, comprising: a sound attenuating element; and a stem; wherein the sound attenuating element comprises a flange which extends rearwardly over a portion of the stem; and wherein the sound attenuating element and the stem are integrally formed of a resilient compressible material, in particular the resilient compressible material is an extruded foam material. It is described that foamed materials may be of an open or closed cell material, and that in a more preferred embodiment at least about 65% by volume of the overall cell volume will include closed cells. WO 2007/0044766 also describes a method of manufacturing an earplug comprising: forming a hollow tube of a resilient compressible material (in particular said forming the hollow tube comprising extruding the tube of a foam material); permanently deforming the hollow tube to form a closed solid portion; allowing an open portion of the hollow tube to remain adjacent to the closed portion; pivoting the open portion outwardly and over the closed portion to delimit a sound attenuating element and a stem. It is described that the resulting closed portion has a greater density than the open portion and that the closed portion forms the stem of the earplug when the open portion is hinged backward into the sound attenuating position.
Although push-to-fit type earplugs exhibit desirable characteristics in various applications, they may be costly and may pose difficult manufacturing challenges.

**Summary**

The present invention provides an earplug including a body having a sound attenuating portion and a semi-rigid stem portion having a relatively greater stiffness than the sound attenuating portion. The body is made entirely of a closed-cell, slow-recovery foam, the sound attenuating portion having a first density $\rho 1$ and the stem portion having a second density p2, and p2 is greater than 1.5 $\rho 1$ (i.e. $\rho 2 > 1.5 \rho 1$). The sound attenuating portion is chemically bonded to the stem portion.
In some embodiments, the present disclosure provides methods of making an earplug described herein, which are two-shot molding processes as set out in claims 12 and 13.
The above summary is not intended to describe each disclosed embodiment or every implementation. The Figures and the Detailed Description, which follow, more particularly exemplify illustrative embodiments.

**Brief Description of Drawings**

The disclosure may be further explained with reference to the appended Figures, wherein like structure is referred to by like numerals throughout the several views, and wherein:

FIG. 1 is a perspective view of an exemplary push-to-fit type earplug according to the present disclosure.
FIG. 2 is cross-sectional view of an exemplary push-to-fit type earplug according to the present disclosure.
FIGS. 3A - 3D are perspective views of exemplary push-to-fit type earplugs according to the present disclosure showing sound attenuating portions having various exemplary shapes.

FIGS. 4A and 4B are cross-sectional views of exemplary mold components that may be used in forming an earplug as described herein.

FIG. 5 is a cross-sectional view of an exemplary mold that may be used in forming an earplug as described herein, in particular in an exemplary method of making a push-to-fit type earplug in a single-shot molding process, such a method not being part of the claimed methods.

This disclosure presents the disclosed subject matter by way of representation and not limitation. It should be understood that numerous other modifications and embodiments can be devised by those skilled in the art which fall within the scope of this disclosure.

## Detailed Description

An earplug that provides hearing protection for a user, and a method of making an earplug, is provided in the present disclosure. An earplug according to the present disclosure includes a body having a sound attenuating portion and a semi-rigid stem portion having a relatively greater stiffness than the sound attenuating portion. The body is made of a closed cell, slow-recovery foam. The density of the sound attenuating portion may be relatively lower than the density of the stem portion, such that at least part of the sound attenuating portion may be comfortably inserted into the ear canal of a user and the relatively stiffer stem portion may be grasped by a user to handle the earplug. The sound attenuating portion has a first density $\rho1$ and the stem portion has a second density $\rho2$, and $|\rho2 > 1.5\, \rho1|$.

The present disclosure further provides methods of making an earplug that minimizes difficult and expensive manufacturing techniques while providing a push-to-fit type earplug that may be easily handled and comfortably worn. Methods include providing a mold having a first unvented cavity in the form of a stem and a second vented cavity in the form of a sound attenuating portion. Methods include two-shot molding processes. As detailed infra, in exemplary embodiments, an exemplary earplug as described herein may be made by a two-shot molding process including the steps of dispensing foamable material into an unvented cavity of a mold, partially curing the foamable material in the unvented cavity, dispensing foamable material into a vented cavity of the mold, and curing the foamable materials in the unvented and vented cavities, wherein as the foamable materials cure, a chemical bond is formed between them to form a push-to-fit type earplug including a compliant sound attenuating portion and a relatively stiffer and/or denser stem portion, and in other exemplary embodiments, a sound attenuating portion may be formed in a first shot and the stem portion formed in a second shot. Upon curing the materials, a push-to-fit type earplug is formed including a sound attenuating portion having a first average density and the stem portion having a second average density that is greater than 1.5 times the first average density.

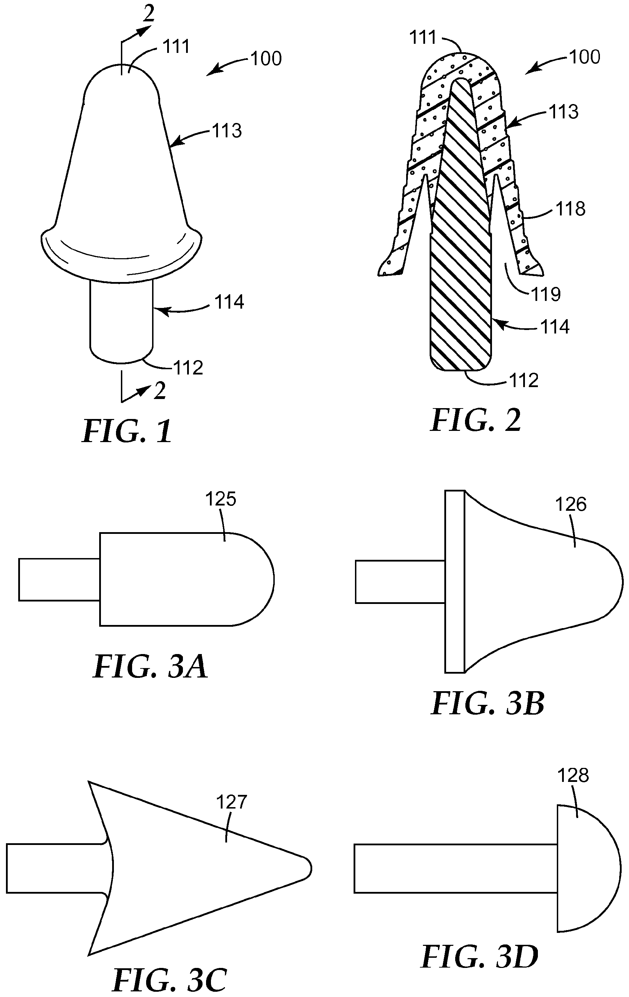

Figures 1 and 2 show an exemplary push-to-fit type earplug 100 according to the present disclosure. In an exemplary embodiment, earplug 100 includes a body made entirely of closed cell foam. The body has a first end 111 and a second end 112, and includes a sound attenuating portion 113 and a semi-rigid stem portion 114. Stem portion 114 is partially surrounded by sound attenuating portion 113, and is partially exposed to allow a user to grasp stem portion 114 to handle earplug 100 and to facilitate insertion of earplug 100 into an ear canal of a user. Stem portion 114 does not extend to first end 111 and only sound attenuating portion 113 is present at first end 111. Only stem portion 114, and not sound attenuating portion 113, is present at second end 112. That is, earplug 100 includes a leading end section formed entirely of less stiff and/or less dense foam, a rear section formed entirely of stiffer and/or denser foam, and an intermediate section including stiffer and/or denser foam covered by the less stiff and/or less dense foam.

In an exemplary embodiment, sound attenuating portion 113 has a tapered or cone shape, and has a diameter at its widest point that is greater than a diameter of stem portion 114. In various other embodiments shown in Figures 3A through 3D, for example, sound attenuating portions 125, 126, 127, 128, respectively, may be hemisphere-shaped, bullet-shaped, bell-shaped, mushroom-shaped, or otherwise shaped to provide a desired fit or to suit a particular application.

During insertion of earplug 100, stem portion 114 provides a handle which may be gripped by a user. Earplug 100, and specifically sound attenuating portion 113, is brought proximate to the user's ear and inserted into the ear canal. Sound attenuating portion 113 compresses as it is positioned, and stem portion 114 provides sufficient stiffness to facilitate insertion. In use, sound attenuating portion 113 is positioned substantially within an ear canal to block the passage of sound and stem portion 114 extends outwardly from the ear canal to provide a handle to remove the earplug.

In various exemplary embodiments, sound attenuating portions 113 may include a flange 118 extending outwardly and defining a flange cavity 119. Flange 118 may collapse inwardly into flange cavity 119 upon insertion into an ear canal of a user.

In an exemplary embodiment, stem portion 114 exhibits relatively greater rigidity or stiffness than sound attenuating portion 113, without introducing hard edges or surfaces that may reduce comfort of a wearer of earplug 100. Stem portion 114 provides sufficient rigidity that earplug 100 may be positioned for use at least partially in the ear of a user by pushing sound attenuating portion 113 into the ear canal with an appropriate force. That is, earplug 100 having a sufficiently stiff stem portion 114 combined with an appropriate sound attenuating portion 113 allows earplug 100 to be positioned for use at least partially in the ear of a user without the need to first compress or "roll down" sound attenuating portion 113. Direct insertion without the need to first compress or "roll down" sound attenuating portion 113, for example, promotes hygiene by limiting contact with sound attenuating portion 113 prior to placement in the ear. Stem portion 114 also exhibits an appropriate level of flexibility such that it may slightly deform to the contours of the ear canal when positioned for use, as discussed in greater detail herein.

The body is made entirely of a closed-cell, slow-recovery foam. Accordingly, sound attenuating portion 113 and stem portion 114 are both made of a slow-recovery foam. Slow recovery foam does not immediately return to its original shape after compression, but rather expands relatively slowly to its original shape. A stem portion made of slow recovery foam, and not including an additional non-foam stiffener element, provides a unique feel to a user handling the earplug. Further, a stem portion made of slow recovery foam is able to flex, bend and compress, for example, when a portion of earplug 100 is inserted into an ear canal of a user. As a result, a stem portion made of a slow-recovery foam, as opposed to a solid plastic, elastomeric, rubber or non-foam material, for example, may provide a more comfortable fit as perceived by a user of earplug 100. Additionally, an earplug 100 including a sound attenuating portion 113 and stem portion 114 made of a slow recovery foam may provide the ability of both sound attenuating portion 113 and stem portion 114 to compress when inserted into an ear canal, thus better conforming to a user's ear canal. Such an earplug is more likely to remain comfortable in a user's ear canal, even during prolonged periods of use.

Creep compliance measures the evolution of strain with time at a constant load. Greater compliance suggests an increased propensity of material to conform under a load. A slow recovery foam that is sufficiently stiff to be pushed into an ear canal while exhibiting creep compliance within an appropriate range is able to at least slightly conform to an ear canal of a user and may result in greater perceived comfort by a user, especially when used for longer periods of time. For example, the combination of a stem portion 114 made entirely of slow recovery foam and having such creep compliance values may provide a particularly desirable fit.

Stem portion 114 may be made of one or more materials and processes resulting in a specified hardness. A desired hardness may depend on the dimensions of stem portion 114 such that stem portion 114 exhibits a desired stiffness. For example, an earplug 100 exhibiting relatively high hardness values in combination with slow recovery foam properties, for example, provides a unique feel to a user when handling the earplug and upon insertion into an ear canal.

Sound attenuating portion 113 and stem portion 114 are made from one or more foam materials that can suitably bond to, and are otherwise compatible with, one another. Sound attenuating portion 113 and stem portion 114 are joined by a chemical bond. The terms "chemical bonding" or "chemically bonded" refer to physical phenomena responsible for the attractive interactions between atoms and molecules. Such bonds include covalent and ionic bonds, as well as hydrogen and Van der Waal's bonds and can depend on the manufacturing process, chemical composition and available functional groups of the foam of sound attenuating portion 113 and/or stem portion 114, as discussed in greater detail herein. In an exemplary embodiment, chemical bonding between foam sound attenuating portion 113 and foam stem portion 114 is facilitated by the use of identical or chemically similar compositions, and the materials of sound attenuating portion 113 and stem portion 114 are selected such that the primary source of bonding between the foam sound attenuating portion 113 and foam stem portion 114 is chemical bonding. An additional adhesive is not required to bond sound attenuating portion 113 and stem portion 114, and such an adhesive is not present between sound attenuating portion 113 and stem portion 114 in an exemplary embodiment.

In some exemplary embodiments, sound attenuating portion 113 and stem portion 114 are formed from a composition including a blend of polyether polyurethane and an acrylic polymer, such as Hypol 2000 available from The Dow Chemical Company and Encor 154S available from Arkema, Inc. In other exemplary embodiments, sound attenuating portion 113 may include a blend of polyether polyurethane and an acrylic polymer and stem portion 114 may be formed from a material composition including a diisocyanate and a polyol, such as Modur PF available from Bayer, Corp. Other suitable materials for forming sound attenuating portion 113 may include foamed thermoplastic resins, and other soft, pliable foams that may be comfortably positioned in an ear canal of a user. Other suitable materials for forming stem portion 114 may include foamed thermoplastic resins, and other suitable materials that may be foamed to exhibit an appropriate stiffness such that sound attenuating portion 113 of earplug 100 may be easily inserted into the ear canal of a user while allowing a desired level of compliance to provide a comfortable fit. In various exemplary embodiments, various additional materials may be included, such as pigments, cell regulators, deionized water, and/or other suitable materials as known in the art. The resulting material is a hydrophilic or hydrophobic, slow recovery, and dynamically stiff foam.

In an exemplary embodiment, stem portion 114 has a generally circular or rounded cross-section such that stem portion 114 exhibits a generally cylindrical shape. A circular cross section may minimize edges that may cause discomfort by contacting portions of a user's ear. In various other exemplary embodiments, elongate core may have a triangular, square, or other suitable cross-section, or may have a cross-section that varies along the length of earplug 100.

In some exemplary embodiments, earplug 100 defines a channel. Earplug 100 having a body defining a channel may be manufactured such that components of a receiver or of a communication system, for example, may be attached to earplug 100. Alternatively or in addition, a channel may accommodate one or more filters or other passive hearing elements to provide an attenuation curve having a desired shape. For example, filters positioned in a channel may cause nonlinear attenuation of high level impulses produced by explosions, gunfire, or the like. A channel may also provide a recess that a cord may be attached to, such that first and second earplugs may be joined, or that ends of a headband may be attached to in a semi-aural hearing protector.

Materials of sound attenuating portion 113 and/or stem portion 114 may be selected to control the friability of the sound attenuating portion 113 and stem portion 114 such that they may not easily be broken or disintegrate during use. The friability of an earplug may be controlled in part by selecting a material having an appropriate molecular weight, with higher molecular weight generally resulting in a less friable earplug. In an exemplary embodiment, sound attenuating portion 113 and stem portion 114 includes a foam polymer having a molecular weight between 100,000 Daltons and 200,000 Daltons, as measured by gel permeation chromatography analysis as known in the art, such as according to ASTM D6474 - 99.

The density of sound attenuating portion 113 and stem portion 114 can be controlled during manufacturing to provide a specified density as desired for a particular application and to control the density of sound attenuating portion 113 as compared to stem portion 114. Sound attenuating portion 113 and stem portion 114 may exhibit densities that vary slightly at different locations, for example, such that sound attenuating portion 113 has an integral outer skin that is denser than the remainder of sound attenuating portion 113. Such a skin may be present on one or both of sound attenuating portion 113 and stem portion 114. Alternatively, sound attenuating portion 113 and stem portion 114 may have substantially uniform densities. In an exemplary embodiment, irrespective of the presence of an integral outer skin or varying densities within sound attenuating portion 113 and stem portion 114, sound attenuating portion 113 has a first density $\rho 1$ and the stem portion has a second density p2. First and second densities $\rho 1$ and p2 can be found by averaging the densities at each location of sound attenuating portion 113 or stem portion 114. In some cases, first and second densities can be approximated by measuring mass and volume of sections or portions of sound attenuating portion 113 and stem portion 114 to calculate first and second densities $\rho 1$ and p2.

The density provides an indication of the ability of sound attenuating portion 113 or stem portion 114 to compress or otherwise conform when subjected to an external force. First average density $\rho 1$ of sound attenuating portion 113 is selected such that sound attenuating portion may provide a comfortable fit by conforming to the ear canal of a user, while providing a desired level of sound attenuation. In various exemplary embodiments, the first average density $\rho 1$ of a sound attenuating portion 113, comprising a polyurethane foam for example, is between 100 kg/m$^3$ and 250 kg/m$^3$, or 110 kg/m$^3$ and 160 kg/m$^3$, or may be about 125 kg/m$^3$. The second average density p2 of stem portion 114 is greater than the first average density $\rho 1$, and in various exemplary embodiments is between 100 kg/m$^3$ and 400 kg/m$^3$, 225 kg/m$^3$ and 275 kg/m$^3$, or may be about 250 kg/m$^3$. The second average density p2 of stem portion 114 is greater than 1.5 times the first average density $\rho 1$ of sound attenuating portion 113 (i.e. | p2 > 1.5 $\rho 1$|). In some cases, the second average density p2 of stem portion 114 may be 2, 3, 4 or more times the first average density $\rho 1$ of sound attenuating portion 113.

In an exemplary embodiment, sound attenuating portion 113 has a first relative density $\rho 1r$ and stem portion 114 has a second relative density p2r. Relative density refers to the density of a foam (i.e., the bulk density) divided by the density of the solid material forming the cell walls, etc., of the foam. Relative density is therefore generally greater in foams having smaller cells and/or thicker cell walls and lower in foams having larger cells and/or thinner cell walls. A relative density of foam provides an indication of the total cell volume and can provide an indication of a stiffness of foam made of a particular material. That is, for a given material and cell structure, a lower relative density suggests a lighter and/or more flexible foam, while a greater relative density suggests a heavier and/or stiffer foam. A relative density of non-cellular polymers, or other polymers not commonly referred to as foams, may exhibit a relative density of 1 or nearly 1, because the density of the sample and the density of the material of which it is made are equal or approximately equal due to limited, or completely absent, cell volume.

Relative densities of sound attenuating portion 113 and stem portion 114 may be controlled to provide an earplug 100 having desired characteristics. In an exemplary embodiment, sound attenuating portion 113 has a first relative density that is lower than a second relative density of stem portion 114. In an exemplary embodiment, sound attenuating portion 113 has a first relative density $\rho 1r$ of between approximately .03 and .75, .05 and .5, or of about .15. Stem portion 114 has a second relative density p2r of between approximately .04 and 0.9, 0.5 and 0.8, or of about 0.3. Accordingly, second relative density p2r may be greater than $\rho 1r$, or approximately 1.5, 2, 3, 4 or more times first relative density $\rho 1r$.

The modulus and hardness of sound attenuating portion 113 and stem portion 114 can be controlled during manufacturing to provide a specified modulus and/or hardness as desired for a particular application and to control the modulus and/or hardness of sound attenuating portion 113 as compared to stem portion 114. In various exemplary embodiments, a stem portion 114 may have a hardness between approximately 100 kPa and 2500 kPa, 250 kPa and 2000 kPa, or between about 500 kPa and 900 kPa, and a modulus between approximately 1000 kPa and 10,000 kPa, 1500 kPa and 5000 kPa, 1800 kPa to 3000 kPa. In various exemplary embodiments the stem portion is formed from a composition including a diisocyanate and a polyol. In various exemplary embodiments, sound attenuating portion 113 may have a hardness between approximately 50 kPa and 500 kPa, or between about 100 kPa and 200 kPa, and a modulus between approximately 100 kPa and 2000 kPa, 200 kPa and 1500 kPa, or between about 250 kPa and 1000 kPa. Accordingly, an exemplary earplug may have a sound attenuating portion 113 having a first modulus that is 1/10, 1/8, 1/4, 1/2, or other fraction of a second modulus exhibited by a stem portion 114, and may have a sound attenuating portion 113 having a first hardness that is 1/5, 1/4, 1/3, 1/2, or other fraction of a second hardness exhibited by a stem portion 114. Accordingly, a stem portion 114 as described herein may be provided that exhibits a modulus and/or hardness that is greater than a modulus and/or hardness of a sound attenuating portion 113, while both stem portion 114 and sound attenuating portion 113 are made from a closed-cell, slow recovery foam, for example. The above modulus and hardness values have been found by the present inventors to provide a desired level of stiffness such that stem portion 114 may be used as a handle to facilitate insertion of sound attenuating portion 113 at least partially into an ear canal, while providing compliance and conformability such that the earplug may be comfortably worn by a user, particularly during extending periods of use.

The present disclosure provides methods of making an earplug 100, as described above. In general the methods include steps of dispensing a foamable material(s) into a mold, the mold having a first unvented cavity in the form of a stem and a second vented cavity in the form of a sound attenuating portion, and curing the material to form a push-to-fit type earplug including a sound attenuating portion having a first average density and the stem portion having a second average density that is greater than 1.5 times the first average density. Such processes allow an earplug comprising a body made entirely of foam, and in some exemplary embodiments made of a uniform chemical composition, that provides a stiff stem to facilitate insertion and a soft, pliable sound attenuating portion that may be comfortably worn by a user.

In general, an earplug is formed in a dispensing molding process in which a mold cavity forming the stem portion is unvented, for example is not vented or has limited venting, to result in a relatively stiffer and/or denser stem portion, while a mold cavity forming the sound attenuation portion is allowed to vent or provides relatively greater venting to result in a relatively less stiff and/or less dense sound attenuating portion. Accordingly, a body is formed having a sound attenuating portion with a stiffness and/or density that is lower than the stiffness and/or density of a stem portion. Such a process allows an earplug made entirely of foam, and in some cases made of a uniform chemical composition, that provides a stiff stem to facilitate insertion and a soft, pliable sound attenuating portion that may be comfortably worn by a user.

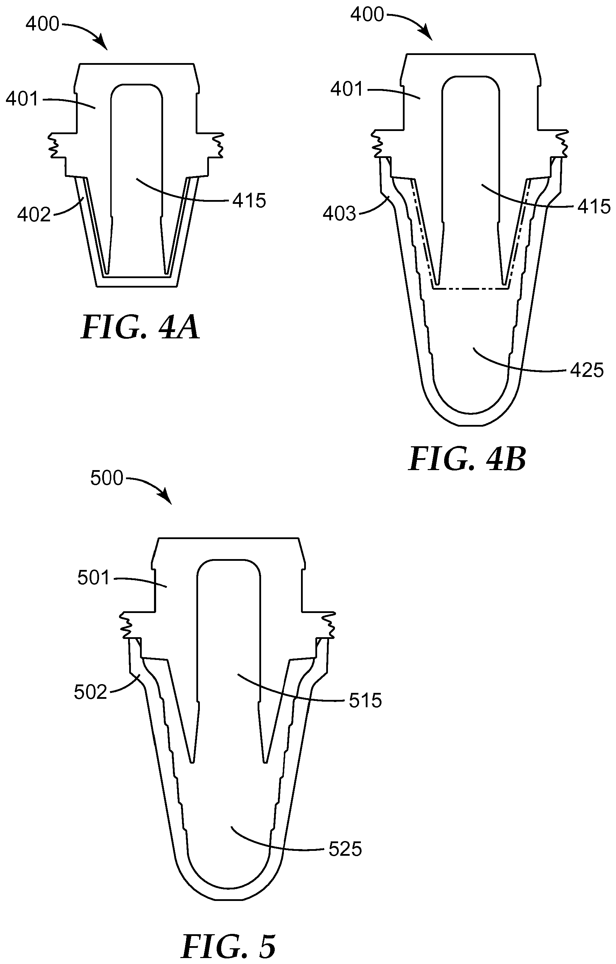

An earplug of the present invention may be made by a two-shot molding process including the steps of dispensing foamable material into an unvented cavity of a mold, partially curing the foamable material in the unvented cavity, dispensing foamable material into a vented cavity of the mold, and curing the materials in the unvented and vented cavities to form a push-to-fit type earplug including a compliant sound attenuating portion and a relatively stiffer and/or denser stem portion. In an exemplary embodiment, a mold 400 includes mold inserts 401, 402, and 403. Mold inserts 402 and 403 may be interchangeable or used in combination to provide a stem cavity 415 and a sound attenuating cavity 425, respectively. In a first configuration shown in Figure 4A, a stem cavity 415 is defined by mold inserts 401 and 402. In a second configuration shown in Figure 4B, a sound attenuating portion cavity 425 is defined by mold insert 401, mold insert 403, and/or a portion of a foamed material in stem cavity 415.

In an embodiment, a foamable material is dispensed into a volume of stem cavity 415 defined by mold insert 401 in a first shot. Mold insert 402 is then clamped to mold insert 401 to form a substantially closed stem cavity 415 that provides limited or no venting. The foamable material is allowed to partially cure, and as applicable at least partially expand or form, before mold inserts 401 and 402 are separated. In a second shot, a foamable material is dispensed into a volume of a sound attenuating portion cavity 425 defined by mold insert 403, for example, and mold insert 403 is clamped to mold insert 401 to form a vented sound attenuating portion cavity 425. Excess gas is allowed to escape from cavity 425, out of the mold and/or partially into stem cavity 415, as the foamable material expands. In various exemplary embodiments, cavity 425 has a total vent area between $0.05 \text{ mm}^2$ to $3.25 \text{ mm}^2$, $0.45 \text{ mm}^2$ to $1.25 \text{ mm}^2$, or about $0.75 \text{ mm}^2$. The foamable materials in the stem cavity 415 and the sound attenuating portion cavity 425 are allowed to cure and, as applicable expand or form, before mold inserts are separated. As the foamable materials cure, a chemical bond is formed between materials in sound attenuating portion cavity 425 and material in stem cavity 415.

In other exemplary embodiments, a sound attenuating portion may be formed in a first shot and the stem portion formed in a second shot. In some exemplary embodiments, stem portion is formed and chemically bonded to a sound attenuating portion without being removed or reinserted into a mold cavity, in contrast to traditional insert molding processes, for example.

Mold 400 may be prepared as appropriate for a selected foamable material and as desired to achieve an earplug having selected characteristics. In various exemplary embodiments, mold 400 is warmed to a desired temperature, for example about 45° C, and an interior surface contacted by foamable material may be coated with polypropylene, or other suitable material that facilitates release of a formed earplug.

Figure 5 shows a mold 500 of an exemplary method of making a push-to-fit type earplug in a single-shot molding process, such a single-shot molding method not being part of the claimed two-shot molding methods. Such a single-shot method includes steps of dispensing foamable material into a mold, the mold having a first unvented cavity in the form of a stem and a second vented cavity in the form of a sound attenuating portion, and curing the material to form a push-to-fit type earplug including a compliant sound attenuating portion and a relatively stiffer and/or denser stem portion. As shown in Figure 5, a mold 500 includes mold inserts 501 and 502. Mold inserts 501 and 502 provide a stem cavity 515 and a sound attenuating cavity 525, respectively. Stem cavity 515 is defined at least in part by mold insert 501 and sound attenuating portion cavity 525 is defined by mold insert 501, mold insert 502, and/or a portion of a material in stem cavity 515.

In a single-shot molding process, a foamable material is dispensed into a volume of stem cavity 515 and/or sound attenuating cavity 525 in a first shot. As the foamable material expands, material will be present in both stem cavity 515 and sound attenuating cavity 525. Excess gas is allowed to escape from sound attenuating cavity 525 while stem cavity 515 is unvented, resulting in an elevated pressure relative to at least portions of sound attenuating cavity 525. The foamable material is allowed to at least partially expand, form, and/or cure before mold inserts 501 and 502 are separated. As the foamable material cures, the material in stem cavity 515 forms a relatively stiffer and/or denser stem due to increased pressure while the material in sound attenuating cavity 525 forms a relatively less stiff and/or less dense sound attenuating portion.

## Examples

The operation of embodiments according to the present disclosure will be further described with regard to the following detailed examples. These examples are offered to further illustrate the various specific embodiments and techniques. It should be understood, however, that many variations and modifications may be made while remaining within the scope of the present description.

### Procedure 1: Modulus and Hardness Test

Modulus is the constant of proportionality describing a relationship between stress and strain in the elastic regime. Hardness is the maximum contact pressure sustained by a sample under stress. Modulus and hardness were measured according to the following procedure.

Samples were prepared by sectioning an earplug lengthwise and adhering the uncut surface to a 2.54 cm (1 inch) diameter aluminum puck using HARDMAN DOUBLE/BUBBLE Extra-Fast Setting Epoxy #04001, specified as having a mixed viscosity at 25 °C of 40 Pa-seconds (40,000 cps). Epoxy was positioned around the edges of the sample at a thickness of the height of the sample, while avoiding epoxy on the cut surface of the sample or otherwise infiltrating the body of the sample. Tests were conducted using an AGLINET G200 Nanoindentor in XP mode using a 1 mm spherical ruby probe with the aluminum puck mounted on the sample stage of the nanoindentor such that the exposed cut surface of the sample was flat and normal to the nanoindentor axis. Testing was conducted using an i.) approach distance of 30 $\mu$m with approach velocity of 50 nm/s; ii.) surface find criteria of 100 N/m; iii.) strain rate of 0.5 $^{\wedge}$s$^{-1}$; iv.) depth setpoint of 100 to 250 $\mu$m; and v.) dwell time after peak load of 10 seconds.

The following equations were used to determine Modulus and Hardness, with E = Reduced Modulus (kPa); H = Hardness (kPa); S = Stiffness (N/m); Pmax = Maximum Load (N); hmax = Maximum Displacement (m); R = Radius of Indenter (m); and A = Contact Area (m$^2$).

$$E = \frac{\sqrt{\pi}}{2} \frac{S}{\sqrt{A}}$$

$$H = \frac{P_{\max}}{A}$$

$$A = 2\pi R \left[ h_{\max} - 0.75 \frac{P_{\max}}{S} \right]$$

### Examples 1 - 3

The earplugs of Examples 1 through 3 were prepared in a two-shot molding process using a mold shown and described with respect to Figures 4A and 4B. A mixture including 86.5 parts Hypol 2000 polyether polyurethane prepolymer, available from The Dow Chemical Co., 73.6 parts Encor 154S acrylic latex material, available from Arkema, Inc., 19.0 parts water based pigment available from DayGlo Color Corporation, and 4.0 parts surfactant cell regulators was prepared and dynamically mixed using a dynamic high shear mixer at an average rate of between 10 revolutions/grams of material pumped and 30 revolutions/grams of material pumped.

Molds were prepared by applying an H-15-1N mold release available from Releasagen Manufacturing Inc. and heating the molds to approximately 45°C. Mixed material was dispensed into sound attenuating portion cavity and a mold top was placed on the mold to provide a vented cavity in the form of a sound attenuating portion. After partially curing, the mold top was removed and replaced with a mold insert to form an unvented stem cavity. A second mixture including 99.5 parts Hypol 2000 polyether polyurethane prepolymer, available from The Dow Chemical Co., 73.6 parts ENCOR 154S acrylic latex, available from Arkema, Inc., 19.0 parts water based pigment available from DayGlo Color Corporation, and 4.0 parts surfactant cell regulators, were dispensed into the stem cavity and allowed to blow, form and cure. The cured earplug was extracted from the mold and dried. Modulus and hardness data was collected according to Procedure 1 at a central stem location approximately at a middle of the cut surface, and at a sound attenuating location on the cut surface. Results are reported in Table 1 below.

**Examples 4 - 6**

The earplugs of Examples 4 through 6 were prepared in a two-shot molding process using a mold shown and described with respect to Figures 4A and 4B. A mixture including 39 parts Mondur PF diisocyanate prepolymer available from Bayer Corporation, 50 parts blend of polyols including ARCOL PPG 425, ARCOL LHT 240, and MULTRANOL 9187, available from Bayer Material Science, and 1 part blend of catalysts including NIAX catalyst A-1, available from Momentive Performance Materials, and BICAT V and BICAT Z, available from Shepard Chemical Company, 3 parts powder pigment available from DayGlo Color Corporation, and 7 parts cell surfactants was dynamically mixed using a dynamic high shear mixer at an average rate of between 10 revolutions/grams of material pumped and 30 revolutions/grams of material pumped.

Molds were prepared by applying release coating 7228 mold release available from Huron Technologies Incorporated and heating the molds to approximately 45°C. Mixed material was dispensed into the stem cavity and a mold top was placed on the mold to provide a closed, non-vented cavity in the form of a stem. After partially curing, the mold top was removed and replaced with a mold insert to form a sound attenuating portion cavity. The dynamically mixed material was dispensed into the cavity and allowed to blow, form and cure while venting of the sound attenuating portion cavity allows some gas to escape. The cured earplug was extracted from the mold and dried. Modulus and hardness data was collected according to Procedure 1 at a central stem location approximately at a middle of the cut surface, and at a sound attenuating location on the cut surface. Results are reported in Table 1 below.

**Comparative Examples A - D**

The earplugs of Comparative Examples A through D were 3M E-A-R PUSH-INS SOFTOUCH uncorded foam earplugs available from 3M Company of St. Paul, Minnesota. Modulus and hardness data was collected at a central stem location approximately at a middle of the cut surface. Results are reported in Table 1 below.

**Comparative Example E - G**

The samples of Comparative Examples E through G were standard EPDM 4235-E foam available from American National Rubber Company. The foam was in the shape of a 15 mm diameter puck having a thickness of 9 mm. The puck was secured to the nanoindentor sample stage and modulus and hardness data was collected according to Procedure 1. Results are reported in Table 2 below.

**Table 1**

|  | Sound Attenuating Portion | | Stem | |
|---|---|---|---|---|
|  | E-(Kpa) | H-(Kpa) | E-(Kpa) | H-(Kpa) |
| Example 1 | 731 | 199 | 1984 | 554 |
| Example 2 | 979 | 174 | 1887 | 544 |
| Example 3 | 959 | 194 | - | - |
| Mean Example 1 - 3 | 890 | 189 | 1935 | 549 |
| Example 4 | 365 | 131 | 2530 | 710 |
| Example 5 | 321 | 115 | 2730 | 812 |
| Example 6 | 289 | 115 | 2640 | 720 |
| Mean Example 4 - 6 | 325 | 120 | 2633 | 747 |
| Comparative Example A | - | - | 258000 | 3900 |
| Comparative Example B | - | - | 260711 | 4090 |
| Comparative Example C | - | - | 258255 | 3235 |

(continued)

| | Sound Attenuating Portion | | Stem | |
|---|---|---|---|---|
| | E-(Kpa) | H-(Kpa) | E-(Kpa) | H-(Kpa) |
| Comparative Example D | - | - | 272650 | 3639 |
| Mean A - D | - | - | 262404 | 3716 |

**Table 2**

| | E-(Kpa) | H-(Kpa) |
|---|---|---|
| Comparative Example E | 621 | 148 |
| Comparative Example F | 592 | 143 |
| Comparative Example G | 566 | 122 |
| Mean F - G | 593 | 138 |

Modulus and Hardness values of Table 1 suggest the earplugs of Examples 1 through 6 have greater compliance at the sound attenuating portions as compared to the stem portions, and the stem portions of Examples 1 through 6 have significantly greater compliance as compared to the stem portions of the earplugs of Comparative Examples A through D.

The foregoing detailed description have been given for clarity of understanding only. No unnecessary limitations are to be understood therefrom. It will be apparent to those skilled in the art that many changes can be made in the embodiments described without departing from the scope of the disclosure. Thus, the scope of the present disclosure should not be limited to the exact details and structures described herein, but rather by the structures described by the language of the claims.

**Claims**

1. **An earplug** (100) comprising:

   a body comprising a sound attenuating portion (113, 125, 126, 127, 128) and a semi-rigid stem portion (114) having a relatively greater stiffness than the sound attenuating portion;
   wherein the body is made entirely of a closed-cell, slow-recovery foam, the sound attenuating portion (113, 125, 126, 127, 128) having a first density $\rho 1$ and the stem portion (114) having a second density p2, and $| p2 > 1.5\,\rho 1|$, and wherein the sound attenuating portion (113, 125, 126, 127, 128) is chemically bonded to the stem portion (114).

2. The earplug (100) of claim 1, wherein the sound attenuation portion (113, 125, 126, 127, 128) has a first relative density $\rho 1r$ and the stem portion (114) has a second relative density p2r, and $|\rho 2r < 0.50|$, wherein relative density is the density of the foam of the respective portion divided by the density of the solid material forming the cell walls of the foam of the respective portion.

3. The earplug (100) of claim 1, wherein the body comprises a first end (111) and a second end (112) and wherein only the sound attenuating portion (113, 125, 126, 127, 128) is present at the first end (111) and only the stem portion (114) is present at the second end (112).

4. The earplug (100) of claim 1, wherein the sound attenuating portion (113, 125, 126, 127, 128) has a front end and a rear end, and the rear end is positioned between 3 mm and 18 mm from the second end (112) of the earplug (100).

5. The earplug (100) of claim 1, wherein the stem portion (114) includes an exposed surface graspable by a user during insertion of a portion of the earplug (100) into an ear canal.

6. The earplug (100) of claim 1, wherein an adhesive is not present between the sound attenuating portion (113, 125, 126, 127, 128) and the stem portion (114).

7. The earplug (100) of claim 1, wherein the sound attenuating portion (113, 125, 126, 127, 128) is formed from a first composition including polyether polyurethane and an acrylic polymer.

8. The earplug (100) of claim 1, wherein the stem portion (114) is formed from a second composition including polyether polyurethane and an acrylic polymer.

9. The earplug (100) of claim 1, wherein the sound attenuating portion (113, 125, 126, 127, 128) and the stem portion (114) are formed from first and second compositions, respectively, each including polyether polyurethane and an acrylic polymer.

10. The earplug (100) of claim 1, wherein the stem portion (114) is formed from a second composition including a diisocyanate and a polyol.

11. The earplug (100) of claim 1, wherein the stem portion (114) and the sound attenuating portion (113, 125, 126, 127, 128) comprise different colors.

12. A method of making an earplug (100) according to claim 1, the method being a two-shot molding process including the steps of:

providing a mold (400) having a first unvented cavity (415) in the form of a stem portion (114) and a second vented cavity (425) in the form of a sound attenuating portion (113, 125, 126, 127, 128);
dispensing a first foamable material composition into the second vented cavity (425) of the mold (400), partially curing the first foamable material in the vented cavity (425),
dispensing a second foamable material composition into the first unvented cavity (415) of the mold (400), and curing the foamable materials in the cavities (415, 425), wherein as the foamable materials cure, a chemical bond is formed between them.

13. A method of making an earplug (100) according to claim 1, the method being a two-shot molding process including the steps of:

providing a mold (400) having a first unvented cavity (415) in the form of a stem portion (114) and a second vented cavity (425) in the form of a sound attenuating portion (113, 125, 126, 127, 128);
dispensing a second foamable material composition into the first unvented cavity (415) of the mold (400), partially curing the second foamable material in the unvented cavity (415),
dispensing a first foamable material composition into the second vented cavity (425) of the mold (400), and curing the foamable materials in the cavities (415, 425), wherein as the foamable materials cure, a chemical bond is formed between them.

**Patentansprüche**

1. Ein Ohrstöpsel (100), aufweisend:

einen Körper, aufweisend einen schalldämpfenden Abschnitt (113, 125, 126, 127, 128) und einen halbstarren Schaftabschnitt (114), der eine relativ größere Steifigkeit als der schalldämpfende Abschnitt aufweist;
wobei der Körper vollständig aus einem geschlossenzelligen, sich langsam erholenden Schaumstoff besteht, wobei der schalldämpfende Abschnitt (113, 125, 126, 127, 128) eine erste Dichte $\rho 1$ aufweist und der Schaftabschnitt (114) eine zweite Dichte $\rho 2$ aufweist und $|\rho 2 > 1,5\,\rho 1|$, und wobei der schalldämpfende Abschnitt (113, 125, 126, 127, 128) an den Schaftabschnitt (114) chemisch gebunden ist.

2. Der Ohrstöpsel (100) nach Anspruch 1, wobei der Schalldämpfungsabschnitt (113, 125, 126, 127, 128) eine erste relative Dichte $\rho 1r$ aufweist und der Schaftabschnitt (114) eine zweite relative Dichte $\rho 2r$ aufweist und $|\rho 2r < 0,50|$, wobei die relative Dichte die Dichte des Schaumstoffs des jeweiligen Abschnitts geteilt durch die Dichte des festen Materials ist, das die Zellwände des Schaumstoffs des jeweiligen Abschnitts ausbildet.

3. Der Ohrstöpsel (100) nach Anspruch 1, wobei der Körper ein erstes Ende (111) und ein zweites Ende (112) aufweist und wobei nur der schalldämpfende Abschnitt (113, 125, 126, 127, 128) an dem ersten Ende (111) vorhanden ist und nur der Schaftabschnitt (114) an dem zweiten Ende (112) vorhanden ist.

**4.** Der Ohrstöpsel (100) nach Anspruch 1, wobei der schalldämpfende Abschnitt (113, 125, 126, 127, 128) ein vorderes Ende und ein hinteres Ende aufweist und das hintere Ende zwischen 3 mm und 18 mm von dem zweiten Ende (112) des Ohrstöpsels (100) positioniert ist.

**5.** Der Ohrstöpsel (100) nach Anspruch 1, wobei der Schaftabschnitt (114) eine freiliegende Oberfläche aufweist, die durch einen Benutzer während einem Einführen eines Abschnitts des Ohrstöpsels (100) in einen Gehörgang gegriffen werden kann.

**6.** Der Ohrstöpsel (100) nach Anspruch 1, wobei zwischen dem schalldämpfenden Abschnitt (113, 125, 126, 127, 128) und dem Schaftabschnitt (114) kein Kleber vorhanden ist.

**7.** Der Ohrstöpsel (100) nach Anspruch 1, wobei der schalldämpfende Abschnitt (113, 125, 126, 127, 128) aus einer ersten Zusammensetzung ausgebildet ist, die Polyetherpolyurethan und ein Acrylpolymer enthält.

**8.** Der Ohrstöpsel (100) nach Anspruch 1, wobei der Schaftabschnitt (114) aus einer zweiten Zusammensetzung ausgebildet ist, die Polyetherpolyurethan und ein Acrylpolymer enthält.

**9.** Der Ohrstöpsel (100) nach Anspruch 1, wobei der schalldämpfende Abschnitt (113, 125, 126, 127, 128) und der Schaftabschnitt (114) aus einer ersten beziehungsweise einer zweiten Zusammensetzung ausgebildet sind, die jeweils Polyetherpolyurethan und ein Acrylpolymer enthalten.

**10.** Der Ohrstöpsel (100) nach Anspruch 1, wobei der Schaftabschnitt (114) aus einer zweiten Zusammensetzung ausgebildet ist, die ein Diisocyanat und ein Polyol enthält.

**11.** Der Ohrstöpsel (100) nach Anspruch 1, wobei der Schaftabschnitt (114) und der schalldämpfende Abschnitt (113, 125, 126, 127, 128) unterschiedliche Farben aufweisen.

**12.** Ein Verfahren zum Herstellen eines Ohrstöpsels (100) nach Anspruch 1, wobei das Verfahren ein Zweistufenspritzgießprozess ist, der die Schritte einschließt:

Bereitstellen einer Form (400), die einem ersten unbelüfteten Hohlraum (415) in Form eines Schaftabschnitts (114) und einem zweiten belüfteten Hohlraum (425) in Form eines schalldämpfenden Abschnitts (113, 125, 126, 127, 128) aufweist;
Abgeben einer ersten schäumbaren Materialzusammensetzung in den zweiten belüfteten Hohlraum (425) der Form (400), teilweises Aushärten des ersten schäumbaren Materials in dem belüfteten Hohlraum (425),
Abgeben einer zweiten schäumbaren Materialzusammensetzung in den ersten unbelüfteten Hohlraum (415) der Form (400) und
Aushärten der schäumbaren Materialien in den Hohlräumen (415, 425), wobei, während die schäumbaren Materialien aushärten, eine chemische Bindung zwischen ihnen ausgebildet wird.

**13.** Ein Verfahren zum Herstellen eines Ohrstöpsels (100) nach Anspruch 1, wobei das Verfahren ein Zweistufenspritzgießprozess ist, der die Schritte einschließt:

Bereitstellen einer Form (400), die einem ersten unbelüfteten Hohlraum (415) in Form eines Schaftabschnitts (114) und einem zweiten belüfteten Hohlraum (425) in Form eines schalldämpfenden Abschnitts (113, 125, 126, 127, 128) aufweist;
Abgeben einer zweiten schäumbaren Materialzusammensetzung in den ersten unbelüfteten Hohlraum (415) der Form (400), teilweises Aushärten des zweiten schäumbaren Materials in dem unbelüfteten Hohlraum (415),
Abgeben einer ersten schäumbaren Materialzusammensetzung in den zweiten belüfteten Hohlraum (425) der Form (400) und
Aushärten der schäumbaren Materialien in den Hohlräumen (415, 425), wobei, während die schäumbaren Materialien aushärten, eine chemische Bindung zwischen ihnen ausgebildet wird.

**Revendications**

**1.** Bouchon d'oreille (100) comprenant :

un corps comprenant une partie d'atténuation des sons (113, 125, 126, 127, 128) et une partie de tige (114) semi-rigide ayant une rigidité relativement plus grande que la partie d'atténuation des sons ;

dans lequel le corps est entièrement fabriqué en une mousse à cellules fermées, de récupération lente, la partie d'atténuation des sons (113, 125, 126, 127, 128) ayant une première masse volumique $\rho 1$ et la partie de tige (114) ayant une seconde masse volumique $\rho 2$, et $|\rho 2 > 1,5 \, \rho 1|$, et dans lequel la partie d'atténuation des sons (113, 125, 126, 127, 128) est chimiquement liée à la partie de tige (114).

2. Bouchon d'oreille (100) selon la revendication 1, dans lequel la partie d'atténuation des sons (113, 125, 126, 127, 128) a une première densité relative $\rho 1r$ et la partie de tige (114) a une seconde densité relative $\rho 2r$, et $|\rho 2r < 0,50|$, dans lequel la densité relative est la masse volumique de la mousse de la partie respective divisée par la masse volumique du matériau solide formant les parois cellulaires de la mousse de la partie respective.

3. Bouchon d'oreille (100) selon la revendication 1, dans lequel le corps comprend une première extrémité (111) et une seconde extrémité (112) et dans lequel seule la partie d'atténuation des sons (113, 125, 126, 127, 128) est présente au niveau de la première extrémité (111) et seule la partie de tige (114) est présente au niveau de la seconde extrémité (112).

4. Bouchon d'oreille (100) selon la revendication 1, dans lequel la partie d'atténuation des sons (113, 125, 126, 127, 128) a une extrémité avant et une extrémité arrière, et l'extrémité arrière est positionnée entre 3 mm et 18 mm de la seconde extrémité (112) du bouchon d'oreille (100).

5. Bouchon d'oreille (100) selon la revendication 1, dans lequel la partie de tige (114) comporte une surface exposée saisissable par un utilisateur pendant l'insertion d'une partie du bouchon d'oreille (100) dans un canal auditif.

6. Bouchon d'oreille (100) selon la revendication 1, dans lequel un adhésif n'est pas présent entre la partie d'atténuation des sons (113, 125, 126, 127, 128) et la partie de tige (114).

7. Bouchon d'oreille (100) selon la revendication 1, dans lequel la partie d'atténuation des sons (113, 125, 126, 127, 128) est formée d'une première composition comportant du polyéther polyuréthane et un polymère acrylique.

8. Bouchon d'oreille (100) selon la revendication 1, dans lequel la partie de tige (114) est formée d'une seconde composition comportant du polyéther polyuréthane et un polymère acrylique.

9. Bouchon d'oreille (100) selon la revendication 1, dans lequel la partie d'atténuation des sons (113, 125, 126, 127, 128) et la partie de tige (114) sont formées des première et seconde compositions, respectivement, comportant chacune du polyéther polyuréthane et un polymère acrylique.

10. Bouchon d'oreille (100) selon la revendication 1, dans lequel la partie de tige (114) est formée d'une seconde composition comportant un diisocyanate et un polyol.

11. Bouchon d'oreille (100) selon la revendication 1, dans lequel la partie de tige (114) et la partie d'atténuation des sons (113, 125, 126, 127, 128) comprennent de couleurs différentes.

12. Procédé de fabrication d'un bouchon d'oreille (100) selon la revendication 1, le procédé étant un processus de moulage en deux temps comportant les étapes de :

fourniture d'un moule (400) ayant une première cavité non ventilée (415) sous la forme d'une partie de tige (114) et une seconde cavité ventilée (425) sous la forme d'une partie d'atténuation des sons (113, 125, 126, 127, 128) ;
distribution d'une première composition de matériau moussable dans la seconde cavité ventilée (425) du moule (400), durcissant partiellement le premier matériau moussable dans la cavité ventilée (425),
distribution d'une seconde composition de matériau moussable dans la première cavité non ventilée (415) du moule (400), et
durcissement des matériaux moussables dans les cavités (415, 425), dans lequel lorsque les matériaux moussables durcissent, une liaison chimique est formée entre eux.

13. Procédé de fabrication d'un bouchon d'oreille (100) selon la revendication 1, le procédé étant un processus de moulage en deux temps comportant les étapes de :

fourniture d'un moule (400) ayant une première cavité non ventilée (415) sous la forme d'une partie de tige (114) et une seconde cavité ventilée (425) sous la forme d'une partie d'atténuation des sons (113, 125, 126, 127, 128) ;

distribution d'une seconde composition de matériau moussable dans la première cavité non ventilée (415) du moule (400), durcissant partiellement le second matériau moussable dans la cavité non ventilée (415),

distribution d'une première composition de matériau moussable dans la première cavité non ventilée (425) du moule (400), et

durcissement des matériaux moussables dans les cavités (415, 425), dans lequel lorsque les matériaux moussables durcissent, une liaison chimique est formée entre eux.

**FIG. 1**

**FIG. 2**

**FIG. 3A**

**FIG. 3B**

**FIG. 3C**

**FIG. 3D**

**FIG. 4A**

**FIG. 4B**

**FIG. 5**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 20070044766 A